# EUROPÄISCHE PATENTSCHRIFT

(11) **EP 1 265 631 B1**
(45) Veröffentlichungstag und Bekanntmachung des Hinweises auf die Patenterteilung: **10.05.2006**
(21) Anmeldenummer: 01913372.7
(22) Anmeldetag: 21.03.2001
(51) Int. Cl.: A61K 39/39, A61K 39/395, A61K 39/29

(54) **VERFAHREN ZUR HERSTELLUNG VON VAKZINEN DIE EINE HITZEBEHANDELTE MISCHUNG VON ZUMINDEST EINEM ANTIGEN MIT ZUMINDEST EINEM ADJUVANS ENTHÄLT**
METHOD FOR PRODUCING VACCINES CONTAINING A HEAT-TREATED MIXTURE CONSISTING OF AT LEAST ONE ANTIGEN AND OF AT LEAST ONE ADJUVANT
PROCEDE DE PRODUCTION DE VACCINS COMPRENANT UN MELANGE TRAITE THERMIQUEMENT D'AU MOINS UN ANTIGENE AVEC AU MOINS UN ADJUVANT

(30) Priorität: 21.03.2000 AT 4722000
(43) Veröffentlichungstag der Anmeldung: 18.12.2002
(73) Patentinhaber: Loibner, Hans, Dr., 1230 Wien (AT)
(72) Erfinder: LOIBNER, Hans, Dr., A-1230 Wien (AT); ECKERT, Helmut, CH-4104 Oberwil (CH)
(74) Vertreter: Alge, Daniel
(86) Internationale Anmeldenummer: PCT/AT2001/000080
(87) Internationale Veröffentlichungsnummer: WO 2001/070264

(56) Entgegenhaltungen:
- WO-A-00/41722
- WO-A-01/35989
- WO-A-98/56419
- P. COOPER ET AL.: "Algammulin, a new vaccine adjuvant comprising gamma inulin particles containing alum: preparation and in vitro properties." VACCINE, Bd. 9, Nr. 5, Mai 1991 (1991-05), Seiten 351-357, XP000215822 Guildford, Grossbritannien
- J. KREUTER: "Nanoparticle-based drug delivery systems." JOURNAL OF CONTROLLED RELEASE, Bd. 16, Nr. 1-2, Juni 1991 (1991-06), Seiten 169-176, XP000219660 Amsterdam, die Niederlande
- K. SPEIDEL ET AL.: "Priming of cytotoxic T lymphocytes by five heat-aggregated antigens in vivo: conditions, efficiency, and relation to antibody responses." EUROPEAN JOURNAL OF IMMUNOLOGY, Bd. 27, Nr. 9, September 1997 (1997-09), Seiten 2391-2399, XP001015867 Weinheim, Deutschland

## Beschreibung

Die vorliegende Erfindung betrifft ein Verfahren zur Herstellung von Vakzinen, die zumindest ein Antigen und zumindest ein Adjuvans aufweisen.

Die Schutzimpfung gehört weltweit zu den effizientesten und erfolgreichsten Errungenschaften der modernen Medizin. Aktive und passive Immunisierung von Mensch und Tier gegen verschiedenste Krankheiten werden verwendet, um die weltweiten Erkrankungs- und Todesfälle an einstigen "Geißeln der Menschheit" effizient zu bekämpfen. Besonders die aktive Immunisierung, bei welcher nicht vermehrungsfähige bakterielle oder virale Antigene verabreicht werden, kann als die effizienteste Methode zur Prophylaxe derartiger Krankheiten weltweit angesehen werden.

Da oftmals die Antigene, die für eine Vakzinierung ausgewählt werden, bei der Impfung im Organismus selbst nur kleine oder unzureichende Immunantworten auslösen, so dass kein wirklicher Schutz gegen die Krankheit entsteht, werden für gewöhnlich in einer Vakzine-Formulierung neben dem aktiven, die Immunantwort auslösenden Wirkstoff auch noch immunstimulierende Hilfsstoffe vorgesehen. Daher enthalten nahezu alle gängigen Impfstoffe sogenannte Adjuvantien, mit welchen die Immunantwort auf bestimmte spezifische Antigene verbessert werden soll. So kann beispielsweise durch die Bindung an bestimmte Adjuvantien, wie Aluminiumhydroxid, die Immunanwort, die von vakzinen induziert wird, deutlich verbessert werden.

In Cooper et al., Vaccine, 9 (5) (1991), 351-357 wird ein Vakzin-Adjuvans beschrieben, das aus Gamma-Inulin und Alum besteht. Gamma-Inulin ist ein lineares Polyfructosan mit ca. 30 bis 60 Fructoseeinheiten in Beta-2,1-Bindung, die in der furanosiden Form vorliegen.

Die WO 98/56419 A betrifft Verfahren zur Verhinderung des Fortschreitens von HMFG-assoziierten Tumoren durch Verwendung des anti-idiotypischen Antikörpers 11D10. In diesem Dokument werden verschiedene Möglichkeiten beschrieben, wie dieser Antikörper verabreicht bzw. als Medikament zur Verfügung gestellt werden kann. Der Antikörper 11D10 kann beispielsweise vor der Verabreichung hitzebehandelt werden kann. Die Hitzebehandlung kann bei ungefähr 40 bis 80 °C, vorzugsweise 45 bis 60 °C für eine Zeit zwischen 5 Minuten und 2 Stunden durchgeführt werden kann.

Kreuter, Journal of controlled release, 16 (1-2) (1991), 169-176 betrifft Nanopartikel, die durch Polymerisierung entweder durch Gammabestrahlung oder Hitzebehandlung in Anwesenheit von Kalium- oder Ammonium-Peroxodisulfat hergestellt werden. Gegebenenfalls können derartige Nanopartikel auch in heißem Öl (120 bis 180 °C) emulgiert werden.

In Speidel et al., European Journal of Immunology, 27 (9) (1997), 2391 - 2399 wird beschrieben, dass ein Influenza-Ganzvirus-Präparat, welches bei 100 °C erhitzt oder sogar autoklaviert worden ist, mit niedrigerer Effizienz in der Lage ist, H-spezifische CTL in B6 Mäusen zu primen. Dabei wird erwähnt, dass in einigen Experimenten Aluminium-Hydroxid als Adjuvans verwendet werden kann, welches vor der Injektion dem zu injizierenden Material zugesetzt worden ist.

Im Zuge der vorliegenden Erfindung hat sich aber gezeigt, dass die Bindung der Antigene, insbesondere von Antigenen auf Polypeptidbasis an derartige Adjuvantien labil und unspezifisch ist, so dass unmittelbar nach Verabreichung von derartigen vakzineformulierungen das Antigen vom Adjuvans zum Großteil abgelöst wird und nur mehr ein geringer Prozentsatz an spezifischen Antigen am Adjuvans verbleibt.

Aufgabe der vorliegenden Erfindung ist daher, verbesserte Vakzineformulierungen zur Verfügung zu stellen, die eine veränderte Verbindung von Antigen und derartigem Adjuvans und somit eine verbesserte Immunantwort auslösen können.

Gegenstand der Erfindung ist ein Verfahren zur Verbesserung der Haftung von Antigenen an Adjuvantien, welches dadurch gekennzeichnet ist, dass zumindest ein Antigen mit zumindest einem Adjuvans gemischt wird (werden) und das Gemisch anschließend bei einer Temperatur von mindestens 80°C für eine Zeitdauer von mindestens 5 min in Lösung oder Suspension hitzebehandelt wird. Es hat sich gezeigt, dass bei diesem Prozess die Haftung der Antigene am Adjuvans entscheidend verändert wird, so dass beim Einbringen der erfindungsgemäß hergestellten Vakzine in das zu immunisierende Individuum (Mensch/Tier) kein sofortiger Austausch des Immunantigens mit den am Verabreichungsort vorhandenen Proteinen erfolgt.

Als Antigene werden erfindungsgemäß bevorzugterweise Antigene auf Polypeptidbasis eingesetzt, also Peptid-Antigene bzw. proteinäre Antigene. Derartige Peptid-Antigene weisen eine Länge ab etwa 6 Aminosäuren auf (z.B. 8 bis 11 Aminosäuren), können aber auch aus sehr viel längeren Polypeptidketten bestehen, die beispielsweise das gesamte native antigene Protein oder die betreffend antigenen Proteinuntereinheiten oder Teile oder Derivate davon umfassen.

Bei dem in dem Gemisch enthaltenen Protein-Antigen kann es sich im Prinzip um jedes beliebige Protein handeln, das als Antigen für eine Impfung in Frage kommt. Unter Proteinen werden in der vorliegenden Erfindung Polypeptide von mehr als fünf Aminosäuren verstanden, die zusätzlich noch andere Stoffe kovalent in der Molekülstruktur enthalten können (wie z.B. Zucker, Lipide, Phosphatgruppen). Die Seitengruppen sind nicht auf natürliche Polypeptidmodifikationen beschränkt, sondern können auch artifizielle Modifikationen sein (wie z.B. Polyethylenglykol). In einer bevorzugten Ausführungsform des erfindungsgemäßen Verfahrens ist das Protein-Antigen ein Antikörper.

Hierbei sind die Antigene der vorliegenden Erfindung nicht auf bestimmte Antigene eingeschränkt, so dass virale (z.B. gegen HAV, HBV, HCV oder HIV) und bakterielle Antigene ebenso wie Allergene, Tumorantigene oder Antigene aus eukaryontischen Pathogenen erfindungsgemäß verwendet werden können. Hierbei kommen auch ganze Viruspartikel, Bakterien oder Fragmente davon als Antigen in Frage.

Als Adjuvantien können erfindungsgemäß oberflächenaktive Feststoffe, auf denen Antigene adsorbieren können, verwendet werden. Besonders bevorzugte Adjuvantien, die im Rahmen der vorliegenden Erfindung verwendet werden, sind Adjuvantien auf Aluminiumbasis, beispielsweise Aluminiumphosphat, insbesondere aber Aluminiumhydroxid, welches sich allgemein als Impfstoffadjuvans bewährt hat.

Es hat sich gezeigt, dass mit der vorliegenden Erfindung trotz der Anwendung von relativ hohen Temperaturen auf die Kombination von Adjuvans und Antigen in Suspension oder Lösung selbst komplexe Antigene nicht oder nur unwesentlich in deren antigenem Potential verändert werden, so dass auch empfindliche labile antigene Proteinstrukturen in erfindungsgemäßen Vakzineformulierungen verwendet werden können.

Es hat sich erfindungsgemäß gezeigt, dass die Hitzebehandlung zumindest bei einer Temperatur von 80°C vorgenommen werden muss, um zu den erfindungsgemäßen Effekten zu gelangen. Dies erklärt auch, weshalb der erfindungsgemäße Effekt bei einer herkömmlichen Pasteurisierung, die meist um die 65°C vorgenommen wird, nicht eintritt. Als besonders günstig haben sich bei der Hitzebehandlung die Anwendung von Temperaturen von 90°C bis 130°C, insbesondere von 100°C bis 125°C, erwiesen sowie die Anwendung (bei Temperaturen über dem Lösungssiedepunkt bei Normaldruck) von geschlossenen Systemen, etwa Autoklaviervorrichtungen oder anderen Druckbehältnissen, wobei dann unter Überdruck gearbeitet wird.

Es zeigt sich, dass bei höheren Temperaturen auch kürzere Behandlungszeiten als bei üblicher Pasteurisierung (sogar - bei bestimmten Proteinen, die eine besonders schnelle Bindung an das Adjuvans bei hohen Temperaturen zeigen - unter 5 min) vorgesehen werden können, so dass die Hitzebehandlungen bevorzugterweise während 20 bis 200 min, insbesondere während 30 bis 60 min (vor allem bei höheren Temperaturen) durchgeführt werden können. Üblicherweise werden im Routineprozess die Vakzineformulierungen für 30 min auf 121°C erhitzt. Bei Erniedrigung der Behandlungstemperatur pro 10°C wird man üblicherweise eine Verdopplung der Erhitzungsdauer vorsehen.

Ein "Nebeneffekt" der vorliegenden Erfindung ist, dass während der Hitzebehandlung auch potentiell vorhandene Krankheitserreger, insbesondere humanpathogene Viren, erfolgreich inaktiviert werden können. Da durch die Anwesenheit von Adjuvantien Temperaturen von über 80°C vorgesehen werden können, ohne dass entscheidende Verschlechterungen in der Antigenstruktur eintreten, kann erfindungsgemäß ein auch hinsichtlich seiner Virussicherheit entscheidend verbessertes Vakzin bzw. ein Vakzin, das einen deutlich geringeren Anteil an denaturierten Komponenten enthält, zur Verfügung gestellt werden.

Obgleich bei den erfindungsgemäß vorzusehenden Temperaturen Proteine in Lösung üblicherweise irreversibel denaturiert werden, behält das Antigen/Adjuvans-Gemisch gemäß der vorliegenden Erfindung überraschenderweise seine immunogenen Eigenschaften im Wesentlichen bei und kann nach wie vor als wirksamer Impfstoff eingesetzt werden.

Durch die Hitzebehandlung und die damit verbundene bessere Fixierung an das Adjuvans hat das erfindungsgemäß hergestellte Präparat sowohl in der Herstellung als auch bei der Lagerung und Distribution entscheidende Vorteile. Der Vorteil der höheren Virussicherheit spielt vor allem auch bei Proteinantigenen, die aus humanem oder tierischem Material stammen, eine herausragende Rolle. Durch das erfindungsgemäße Verfahren und die dadurch erfolgende Sterilisierung ist es möglich, die Hitzebehandlung, aber auch die nachfolgenden Verpackungsschritte ohne Konservierungs- oder Stabilisierungsstoffe durchzuführen. Bei Anwendung des erfindungsgemäßen Verfahrens an bereits abgefülltem Impfstoff ist es möglich, durch die erfolgte Sterilisierung Konservierungsstoffe, wie z.B. Thimerosal, in der Formulierung wegzulassen.

Der Begriff "Vakzine" bedeutet im Rahmen der vorliegenden Erfindung ein Agens zur aktiven Immunisierung, d.h. eine Induktion einer spezifischen Immunantwort durch Verabreichung (z.B. subkutan, intradermal, intramuskulär, ev. auch oral, nasal) von geringen Mengen eines Antigens (eines Stoffes, der vom Immunsystem des vakzinierten Individuums als fremd und damit immunogen erkannt wird) in einer geeigneten immunogenen Formulierung. Das Antigen wird also als "Trigger" für das Immunsystem benutzt, um eine für das Antigen spezifische Immunantwort aufzubauen. Die dazu benötigten Mengen des Antigens können grundsätzlich sehr gering sein (manche Impfstoffe beinhalten nur ca. 5-10 µg Antigen pro Impfdosis). Charakteristisch für eine aktive Immunisierung ist eine in großen Bereichen nur wenig Mengen-abhängige Dosis-Wirkungskurve. Das heißt, dass die Immunantwort in einem weiten Dosisbereich in etwa gleich stark ausfällt. Daraus folgt, dass bei einer Vakzinierung der gewünschte Effekt, also die Induktion einer Immunantwort, bereits mit sehr geringen Antigenmengen erzielt werden kann, aber auch mit wesentlich höheren Antigenmengen in vergleichbarer Weise erzielt werden kann. Es ist aber naturgemäß wünschenswert, grundsätzlich mit möglichst niedrigen Dosierungen zu arbeiten, insbesondere im Hinblick auf Nebenwirkungen, Materialkosten etc., was bei einer Impfung zum Tragen kommt.

Vakzine können für verschiedene Zwecke verwendet werden: Zur Etablierung eines derartigen Immunstatus der geimpften Personen oder Tiere, der sie vor pathogenen Mikroorganismen, wie z.B. vor bestimmten Viren oder Bakterien schützt. Dazu gehören auch Impfstoffe gegen Krebs. Dabei soll das Immunsystem von Krebspatienten selektiv aktiviert werden, maligne Zellen zu bekämpfen. Das wird mittels verschiedenster Ansätze versucht. Eine neuartige Methode der Krebsvakzinierung ist in der Anmeldung WO 0041722 beschrieben. Der darin beschriebene monoklonale Antikörper HE2, der als Vakzin-Antigen in einer Krebsimpfung verwendet wird, dient als Beispiel, jedoch nicht auf dieses eingeschränkt, für die Formulierung eines Impfstoffes nach der hier beschriebenen Methode.

Eine Vakzine im Sinne der vorliegenden Erfindung kann grundsätzlich sowohl im therapeutischen Sinne als auch im prophylaktischen Sinne (wie bei allen antimikrobiellen Impfstoffen) verwendet werden.

Die vorliegende Erfindung betrifft auch eine Vakzineformulierung, die erhalten wird oder die erhältlich ist durch das erfindungsgemäße Verfahren, wobei das Adjuvans ein Adjuvans auf Aluminiumbasis, insbesondere Aluminiumphosphat oder Aluminiumhydroxid, ist. Für die bevorzugten Ausführungsformen dieser Vakzineformulierung gilt dabei das oben im Zusammenhang mit dem erfindungsgemäßen Verfahren Gesagte.

Mit der vorliegenden Erfindung lassen sich besonders effektive humane Vakzinformulierungen mit (gegen) Proteine(n) herstellen, von denen man bislang geglaubt hat, dass sie Hitzebehandlungen bei derart hohen Temperaturen, wie sie erfindungsgemäß vorgesehen werden, nicht überstehen. Aber gerade für diese Proteine scheint die kombinierte Hitzebehandlung in Anwesenheit von Adjuvantien keine oder eine nur unwesentliche Beeinträchtigung der immunstimulierenden Eigenschaften mit sich zu bringen, zumindest bleibt die funktionelle Immunantwort, i.e. die erwünschte protektive Wirkung,von der erfindungsgemäßen Hitzebehandlung unbetroffen.

Einer der entscheidenden Vorteile dieser derartigen Vakzinformulierung liegt wie erwähnt in der veränderten Haftung bzw. Bindung des Antigens zum Adjuvans, so dass diese Bindung bei Verabreichung an einen Impfling nicht messbar gelöst wird, selbst bei einem Überschuss von anderen Proteinen am Ort der Verabreichung. Bei herkömmlichen Antigen/Adjuvans-Formulierungen zeigt sich - wie in den nachfolgenden Beispielen gezeigt -, dass es nur etwa 1 min dauert, bis nahezu sämtliches Antigen im zu impfenden Organismus bereits nicht mehr in einer mit dem Adjuvans verbundenen Form vorliegt. Dies kann mit der erfindungsgemäßen Vakzine erfolgreich verhindert werden, so dass eine wesentlich langsamere Desorption von Adjuvans eintritt. Das erfindungsgemäß behandelte Vakzin zeigt dabei eine gegenüber dem nicht behandelten Vakzin zumindest 50%, vorzugsweise zumindest 90%, insbesondere zumindest 99% verbesserte (= verlangsamte) Desorption des Antigens vom Adjuvans (gemessen z.B. 10 min oder 1 h nach Einbringen der Vakzineformulierung in 2% fötale Kälberserum-Lösung).

Die Erfindung wird anhand der Zeichnung sowie der nachfolgenden Beispiele, auf die selbstverständlich nicht eingeschränkt sein soll, näher erläutert.

Es zeigt:

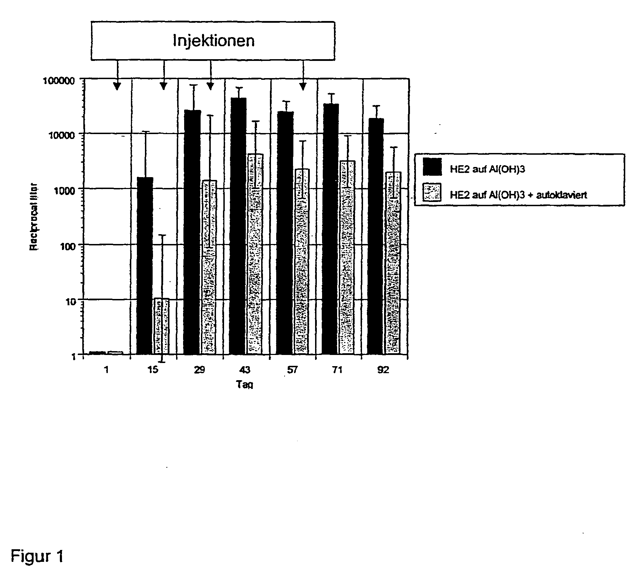

Fig. 1 die Immunantwort, die durch eine Antikörpervakzine, die hitzebehandelt wurde, in Affen erzeugt werden kann (s. Beispiel 1).

### Beispiele:

### Beispiel 1: Hitze-sterilisierte Formulierung des monoklonalen Antikörpers HE2 auf Aluminiumhydroxid

0,5 mg HE2 (Maus-monoklonaler Antikörper IgG₂a) absorbiert auf 1,67 mg Aluminiumhydroxid in 0,5 ml 1 mM Phosphat-Puffer pH 6,0/155 mM NaCl wurde 30 min auf 121°C erhitzt. Vier Rhesusaffen wurden jeweils an den Tagen 1, 15, 29 und 57 subkutan mit dieser Dosis immunisiert. Die Sera von verschiedenen Zeitpunkten wurden mittels ELISA auf Induktion von Antikörpern gegen HE2 getestet. Als Vergleich wurde die gleiche Formulierung, allerdings ohne Hitzebehandlung, geimpft und ebenfalls analysiert (Figur 1). Es ist zu sehen, dass mit der hitzebehandelten Vakzine kaum niedrigere Titer gegen das Protein-Antigen erzeugt wurden.

Die nachfolgende Tabelle zeigt die geringere Desorption der hitzebehandelten Vakzineformulierung im Vergleich zur unbehandelten Formulierung:

Das Serum von immunisierten Affen (jeweils 4 Individuen) wurde im ELISA auf vorhandenes HE2 getestet. Auf den ELISA-Platten wurde affinitätsgereinigtes Ziegen-anti-HE2 Serum immobilisiert. Die Sera wurden in verschiedenen Verdünnungen mit anti-Maus lgG-Peroxidase-Konjugat auf spezifische Antikörper getestet. Die Sensitivität des Tests lag bei 10 ng HE2/ml. Die Proben wurden vor der ersten Immunisierung, sowie 1, 4 und 24 h nach der Immunisierung gezogen. Als externer quantitativer Standard wurde HE2 verwendet. Man kann sehen, dass die hitzebehandelte Vakzineformulierung keinen messbaren HE2-Spiegel im Blut verursachte, während die nicht-hitzebehandelte Vakzineformulierung einen Großteil des HE2 innerhalb von 24 h an das Serum abgibt.

| ***Zeitpunkt*** | ***Hitzebehandelte Vakzine*** | ***nicht behandelte Vakzine*** |
|---|---|---|
| 0 | 0; 0; 0; 0 ng/ml | 0; 0; 0; 0 ng/ml |
| 1 h | 0; 0; 0; 0 ng/ml | 13; 17; 74; |
| | | 280 ng/ml |
| 4 h | 0; 0; 0; 0 ng/ml | 200, 280, 400, |
| | | 740 ng/ml |
| 24 h | 0; 0; 0; 0 ng/ml | 960, 960, 1000, |
| | | 740 ng/ml |

### Beispiel 2: Hitzebehandlung eines Hepatitis B Impfstoffes

EngerixB (SmithKline Beecham; 20 µg/ml) wurde in Stichfläschchen gefüllt und 30 min bei 121°C hitzebehandelt.

Die Immunogenität des so behandelten Impfstoffes wurde in Mäusen getestet. Dazu wurden je 8 Mäuse (Him:OF1, weiblich; 20 bis 25 g Lebendgewicht) mit dem hitzebehandelten bzw. mit dem nicht-behandelten Impfstoff immunisiert (0,155 ml; i.p.), eine weitere Kontrollgruppe wurde nicht immunisiert. Nach 2 Wochen wurde den Mäusen Blut abgenommen und der Hepatitis-spezifische Titer bestimmt.

Dafür wurden 96 well ELISA-Platten (MaxiSorp, Nunc, Dänemark) mit rekombinantem HBs-Antigen, adr Subtyp (Fitzgerald Industries Inc., USA) beschichtet (10 µg/ml Beschichtungspuffer; Dauer: über Nacht, bei 4°C). Nach dem Waschen wurden Verdünnungsreihen der Sera der einzelnen Mäuse (in Waschpuffer) aufgetragen und 8 h bei Raumtemperatur inkubiert. Nach neuerlichem Waschen, wurde mit anti-Maus-Immunglobulin-Peroxidase Konjugat (Sigma, USA) mit entsprechender Verdünnung bei Raumtemperatur inkubiert (2 h). Nach neuerlichem Waschen der Platten wurde Substrat zugegeben, die Färbung wurde nach 30 min gestoppt und im Photometer bei 492 nm gemessen.

Die relative Titerzunahme wurde durch Vergleich der Titer der beiden Impfstoffgruppen mit dem Titer der nicht geimpften Mäuse verglichen. Es zeigte sich, dass die relativen Titeranstiege in den beiden Impfstoffgruppen gleich hoch waren.

### Material:

Beschichtungspuffer: 15 mM Na₂CO₃, 35 mM NaHCO₃, 3 mM NaN₃, pH-Wert: 9,6
PBS: 138 mM NaCl; 1,5 mM KH₂PO₄; 2,7 mM KCl; 6,5 mM Na₂HPO₄; pH-Wert: 7,2
Waschpuffer: 0,05 % Tween 20 in PBS
Färbepuffer: 24,3 mM Zitronensäure; 51,4 mM Na₂HPO₄; pH-Wert: 5,0
Substrat: 40 mg o-Phenylendiamin Dihydrochlorid; 100 ml Färbepuffer; 20 µl H₂O₂ (30%)
Stopplösung: 4 N H₂SO₄

### Beispiel 3: Untersuchung zum Adsorptions- und Desorptionsverhalten des Antigens am Alu-Gel

Die im Beispiel 1 beschriebenen HE2 Formulierungen (hitzedenaturiert und nicht-hitzedenaturiert) werden in einem Verdrängungstest untersucht:

Die Suspension der Impfstoffe wurde abzentrifugiert; der Überstand wurde in einem ELISA auf Anwesenheit des Antigens (spezifisches Maus-Immunglobulin (HE2)) getestet. Im Überstand der nicht-hitzebehandelten Vakzine konnte 0,1% der insgesamt formulierten Antikörpermenge nachgewiesen werden, im Überstand der hitzedenaturierten konnte kein Antigen nachgewiesen werden.

Die abzentrifugierten Impfstoffe wurden jeweils in PBS plus 2% FKS (fötales Kälberserum), aufgeschlämmt und bei 37°C, 1 h geschüttelt.

Danach wurde erneut abzentrifugiert und die jeweiligen Überstände in oben erwähntem ELISA getestet. Im Überstand der nicht-hitzebehandelten Vakzine konnte mehr als 95% der insgesamt formulierten Antikörpermenge nachgewiesen werden, im Überstand der hitzedenaturierten konnte wieder kein Antigen nachgewiesen werden.

Die in diesem Beispiel angeführten Versuche und Ergebnisse zeigen, dass bei der nach dem erfindungsgemäßen Verfahren hergestellten Vakzineformulierung das Antigen (unter physiologischen Bedingungen) irreversibel an das Adjuvans gebunden ist.

### ELISA:

Der ELISA wurde, wenn nicht anders angeführt, nach üblichen Methoden durchgeführt.

Die Inkubationszeiten sind für das Beschichten 30 min, für alle anderen Schritte (Proben; Konjugat) 1 h bei jeweils 37°C. Die verwendeten Volumina sind in allen Schritten 100 µl pro Näpfchen, mit Ausnahme des Blockierens (200 µl pro Näpfchen).

Zum Beschichten der ELISA-Platten wird ein polyklonaler Ziegen-anti-HE2 Antikörper (IGN111) verwendet, der immunaffinitätschromatographisch gereinigt wurde. Beschichtungskonzentration (10 g/ml Beschichtungspuffer).

Als Standard dient HE2 (Mouse IgG 2a, kappa) (10 mg/ml in phosphatgepufferter Salzlösung; pH = 6,0)

Sowohl vom Standard, als auch von den Proben wurden Verdünnungsreihen in PBS mit 2% FKS hergestellt und die einzelnen Verdünnungen aufgetragen.

Zum Nachweis von HE2 aus den Probenverdünnungen und den Standardverdünnungen, der an IGN111 gebunden hat, wurde HRP (Horseradish Peroxidase)-anti-Maus IgG2a (Zymed, USA) als Enzymkonjugat verwendet.

Nach der Enzym-Substratfärbung wurde die Reaktion mit Schwefelsäure gestoppt und die Färbung bei 492 nm gemessen. Zur quantitativen Auswertung wurden die Verdünnungsreihen der Proben mit der Verdünnungsreihe des Standards (HE2) entsprechend verglichen.

### Material:

### Waschpuffer:

| | |
|---|---|
| NaCl | 21,2 g |
| TritonX-100 | 2,5 ml |
| PBS | ad 1000,0 ml |

### Beschichtungspuffer:

| | |
|---|---|
| Na₂CO₃ | 1,59 g |
| NaHCO₃ | 2,93 g |
| Aqua dest. | ad 1000,0 ml |
| pH = 9,6 | |

### Färbepuffer:

| | |
|---|---|
| Zitronensäure | 5,6 g/l |
| di-Na₂HPO₄ x 2H₂O | 9,15 g/l |
| Aqua dest. | ad 1000,0 ml |
| pH = 5,0 | |

### PBS :

| | |
|---|---|
| NaCl | 8,0 g/l |
| KH₂PO₄ | 200,0 mg/l |
| KCl | 200,0 mg/l |
| Na₂HPO₄ | 1,44 g/l |
| Aqua dest. | ad 1000,0 ml |
| pH = 7,2 | |

### FKS (fötales Kälberserum):

Hitzeinaktiviert (1 h bei 56°C),
Gibco Life Technologies Cat. Nr.: 10270-106

Substratlösung: 1 o-Phenylendiamine Dihydrochloride Tablette (Sigma, P-8287) pro 10 ml Färbepuffer Plus 60 µl H₂O₂ 30% (Merck 1.08597.1000).

H₂SO₄ 30%: Fluka, 84724

ELISA Platte: F 96 Maxisorp, Nunc-Immuno Plate, Nunc Brand Products, 442404

## Patentansprüche

1. Verfahren zur Verbesserung der Haftung von Antigenen an Adjuvantien, **dadurch gekennzeichnet, dass** zumindest ein Antigen auf Polypeptidbasia mit zumindest einem Adjuvans gemischt wird und das Gemisch anschließend bei einer Temperatur von über 80°C für eine Zeitdauer von mindestens 5 min hitzebehandelt wird.

2. Verfahren nach Anspruch 1, **dadurch gekennzeichnet, dass** ein oder mehrere Antikörper, insbesondere monoklonale Antikörper, als Antigen (e) verwendet werden.

3. Verfahren nach Anspruch 1 oder 2, **dadurch gekennzeichnet, dass** mehrere verschiedene Antigene mit zumindest einem Adjuvans gemischt werden.

4. Verfahren nach Anspruch 2 oder 3, **dadurch gekennzeichnet, dass** Antikörper mit verschiedenen Spezifitäten als Antigene mit zumindest einem Adjuvans gemischt werden.

5. Verfahren nach einem der Ansprüche 1 bis 4, **dadurch gekennzeichnet, dass** der Antikörper HE2 oder ein Antikörper, der dieselbe Bindungsspezifität wie der Antikörper HE2 aufweist, als Antigen mit zumindest einem Adjuvans gemischt wird.

6. Verfahren nach einem der Ansprüche 1 bis 5, **dadurch gekennzeichnet, dass** die Hitzebehandlung bei einer Temperatur von 90° bis 130°C, insbesondere von 100° bis 125°C, durchgeführt wird.

7. verfahren nach einem der Ansprüche 1 bis 6, **dadurch gekennzeichnet, dass** die Hitzebehandlung während 20 bis 200, insbesondere während 30 bis 60 min durchgeführt wird.

8. Verfahren nach einem der Ansprüche 1 bis 7, **dadurch gekennzeichnet, dass** als Adjuvans ein oberflächenaktiver Feststoff, vorzugsweise auf Aluminiumbasis, insbesondere Aluminiumhydroxid, verwendet wird.

9. Vakzineformulierung, erhältlich nach einem der Ansprüche 1 bis 8, wobei das Adjuvans ein Adjuvans auf Aluminiumbasis, insbesondere Aluminiumphosphat oder Aluminiumhydroxid, ist.

## Claims

1. A method of improving adhesion of antigens on adjuvants, **characterised in that** at least one polypeptide-based antigen is mixed with at least one adjuvant and the mixture subsequently is heat-treated at a temperature of higher than 80°C for a period of time of at least 5 min.

2. A method according to claim 1, **characterised in that** one or more antibodies, in particular monoclonal antibodies, is (are) used as antigen(s).

3. A method according to claim 1 or 2, **characterised in that** several different antigens are mixed with at least one adjuvant.

4. A method according to claim 2 or 3, **characterised in that** antibodies with different specificities are mixed as antigens with at least one adjuvant.

5. A method according to any one of claims 1 to 4, **characterised in that** the antibody HE2 or an antibody which has the same binding specificity as antibody HE2 is mixed as antigen with at least one adjuvant.

6. A method according to any one of claims 1 to 5, **characterised in that** the heat treatment is carried out at a temperature of from 90°C to 130°C, in particular from 100°C to 125°C.

7. A method according to any one of claims 1 to 6, **characterised in that** the heat treatment is carried out for 20 to 200, in particular for 30 to 60 min.

8. A method according to any one of claims 1 to 7, **characterised in that** a surface-active solid substance, preferably based on aluminium, in particular aluminium hydroxide, is used as adjuvant.

9. A vaccine formulation, obtainable according to any one of claims 1 to 8, wherein the adjuvant is an aluminium-based adjuvant, in particular aluminium phosphate or aluminium hydroxide.

## Revendications

1. Procédé pour améliorer l'adhésion d'antigènes à des adjuvants, **caractérisé en ce qu'**au moins un antigène à base polypeptidique est mélangé avec au moins un adjuvant et le mélange est ensuite traité à la chaleur pendant une période de temps d'au moins 5 minutes à une température au-dessus de 80°C.

2. Procédé suivant la revendication 1, **caractérisé en ce qu'**un ou plusieurs anticorps, en particulier des anticorps monoclonaux, est ou sont utilisé(s) comme antigène(s).

3. Procédé suivant la revendication 1 ou 2, **caractérisé en ce que** plusieurs antigènes différents sont mélangés avec au moins un adjuvant.

4. Procédé suivant la revendication 2 ou 3, **caractérisé en ce que** des anticorps de différentes spécificités sont mélangés comme antigènes avec au moins un adjuvant.

5. Procédé suivant l'une des revendications 1 à 4, **caractérisé en ce que** l'anticorps HE2, ou un anticorps qui présente la même spécificité de liaison que l'anticorps HE2, est mélangé comme antigène avec au moins un adjuvant.

6. Procédé suivant l'une des revendications 1 à 5, **caractérisé en ce que** le traitement à la chaleur est conduit à une température de 90 à 130°C, en particulier de 100 à 125°C.

7. Procédé suivant l'une des revendications 1 à 6, **caractérisé en ce que** le traitement à la chaleur est conduit pendant 20 à 200 minutes, en particulier pendant 30 à 60 minutes.

8. Procédé suivant l'une des revendications 1 à 7, **caractérisé en ce que** l'adjuvant utilisé est une substance solide tensioactive, avantageusement à base d'aluminium, en particulier l'hydroxyde d'aluminium.

9. Formulation pour vaccin, pouvant être obtenue suivant l'une des revendications 1 à 8, dans laquelle l'adjuvant est un adjuvant à base d'aluminium, en particulier le phosphate d'aluminium ou l'hydroxyde d'aluminium.
